# EUROPÄISCHE PATENTSCHRIFT

(11) **EP 1 738 161 B1**
(45) Veröffentlichungstag und Bekanntmachung des Hinweises auf die Patenterteilung: **08.07.2015**
(21) Anmeldenummer: 05743344.3
(22) Anmeldetag: 22.04.2005
(51) Int. Cl.: G01N 27/414, G01N 33/00

(54) **VERFAHREN ZUR MINIMIERUNG VON QUEREMPFINDLICHKEITEN BEI FET-BASIERTEN GASSENSOREN**
METHOD FOR MINIMIZING CROSS SENSITIVITY IN FET-BASED GAS SENSORS
PROCEDE POUR MINIMISER DES SENSIBILITES TRANSVERSALES DANS DES CAPTEURS DE GAZ A BASE DE TRANSISTORS A EFFET DE CHAMP

(30) Priorität: 22.04.2004 DE 102004019604
(43) Veröffentlichungstag der Anmeldung: 03.01.2007
(73) Patentinhaber: Micronas GmbH, 79108 Freiburg i. Br. (DE)
(72) Erfinder: FLEISCHER, Maximillian, 85635 Höhenkirchen (DE); LAMPE, Uwe, 21614 Buxtehude (DE); MEIXNER, Hans, 85540 Haar (DE); POHLE, Roland, 85570 Herdweg (DE); SIMON, Elfriede, 80639 München (DE)
(74) Vertreter: Koch Müller Patentanwaltsgesellschaft mbH
(86) Internationale Anmeldenummer: PCT/EP2005/004318
(87) Internationale Veröffentlichungsnummer: WO 2005/103668

(56) Entgegenhaltungen:
- DE-A1- 4 333 875
- PARIS R ET AL: "Low drift air-gap CMOS-FET gas sensor" PROCEEDINGS OF IEEE SENSORS 2002. ORLANDO, FL, JUNE 12 - 14, 2002, IEEE INTERNATIONAL CONFERENCE ON SENSORS, NEW YORK, NY : IEEE, US, Bd. VOL. 1 OF 2. CONF. 1, 12. Juni 2002 (2002-06-12), Seiten 421-425, XP010605129 ISBN: 0-7803-7454-1
- BURGMAIR M ET AL: "Humidity and temperature compensation in work function gas sensor FETs" SENSORS AND ACTUATORS B, ELSEVIER SEQUOIA S.A., LAUSANNE, CH, Bd. 93, Nr. 1-3, 1. August 2003 (2003-08-01), Seiten 271-275, XP004437110 ISSN: 0925-4005
- BURGMAIR M ET AL: "Field effect transducers for work function gas measurements: device improvements and comparison of performance" SENSORS AND ACTUATORS B, ELSEVIER SEQUOIA S.A., LAUSANNE, CH, Bd. 95, Nr. 1-3, 15. Oktober 2003 (2003-10-15), Seiten 183-188, XP004454668 ISSN: 0925-4005
- GERGINTSCHEW Z ET AL: "The capacitively controlled field effect transistor (CCFET) as a new low power gas sensor" SENSORS AND ACTUATORS B, ELSEVIER SEQUOIA S.A., LAUSANNE, CH, Bd. 36, Nr. 1, Oktober 1996 (1996-10), Seiten 285-289, XP004061082 ISSN: 0925-4005

## Beschreibung

Verfahren zur Messung von Gasen und/ oder Minimierung von Querempfindlichkeiten bei FET-basierten Gassensoren.

Die Erfindung betrifft ein Verfahren zur Verbesserung der Selektivität von FET-basierten Gassensoren, wobei störende Einflüsse von Querempfindlichkeiten gemindert werden.

Gassensoren, die die Austrittsarbeitsänderung von sensitiven Materialien als physikalische Größe benutzen und auswerten erfahren in der letzten Zeit ein gesteigertes Interesse. Gründe hierfür sind die Möglichkeiten, diese mit geringen Betriebsenergien betreiben zu können (low-power/geringe Betriebsleistung), sowie eine kostengünstige Fertigungs- und Aufbautechnologie derartiger Gassensoren (low-cost/geringe Herstellungskosten), sowie eine breite Palette von Gasen, die mit *dieser* Plattformtechnologie detektiert werden können (high versatility/Vielseitigkeit). Dabei können zahlreiche unterschiedliche Detektionssubstanzen in derartigen Aufbauten integriert werden. Aufbau und Betriebsverfahren sind beispielsweise aus den Patentanmeldungen [I-III] bekannt: Es können eine Vielzahl von Materialien für sensitive Schichten derartiger Gassensoren verwendet werden.

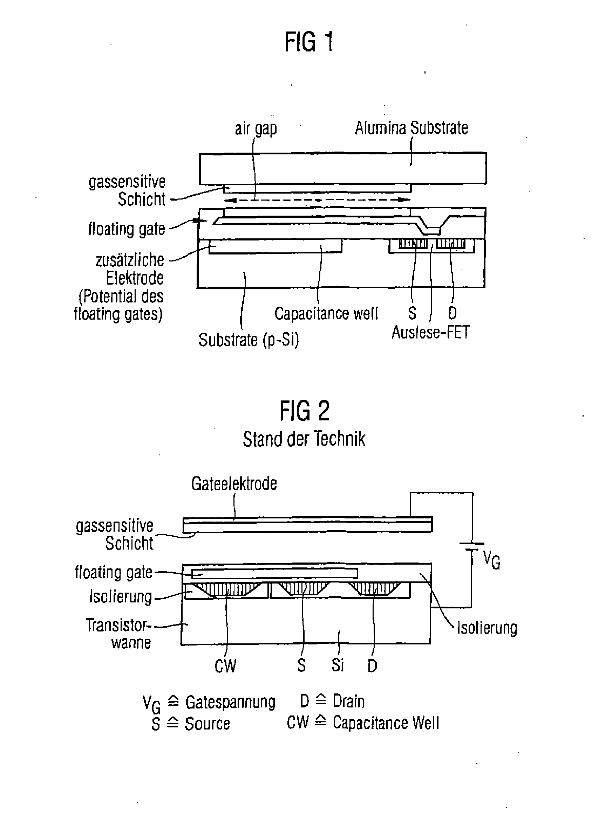

Der Grundaufbau dieser Gassensoren ist in Figur 2 erläutert. Diese zeigt einen schematischen Aufbau von AustrittsarbeitsGassensoren mit FET-Auslesung, insbesondere einen SGFET (Suspended Gate Feldfeffekttransistor, FET mit abgehobener Gateelektrode).

An der sensitiven Schicht, mit der die Unterseite der abgehobenen Gateelektrode beschichtet ist, entsteht bei Präsenz des zu detektierenden Gases ein elektrisches Potential, das der Änderung der Austrittsarbeit des sensitiven Materials entspricht (typischerweise 50-100mV). Dieses wirkt auf den Kanal der FET-Struktur und verändert den Source-Drain-Strom. Ausgelesen wird direkt der geänderte Source-Drain-Strom. Alternativ wird durch Anlegen einer zusätzlichen Spannung an das abgehobene Gate oder an die Transistorwanne die Änderung des Source-Drain-Stromes zurückgestellt. Dabei stellt die zusätzlich angelegte Spannung das Auslesesignal dar, welches mit der Austrittsarbeitsänderung der sensitiven Schicht direkt korreliert.

Ein Grundproblem aller Gassensoren und auch des beschriebenen Typus ist die eingeschränkte Selektivität. D.h. die Sensoren reagieren unter Umständen nicht nur auf das Zielgas, sondern auch auf andere Gase, was mit Querempfindlichkeit bezeichnet wird. Die überlagerten Gassignale führen dabei in manchen Applikation zu einer Situation, bei der die Bestimmung der Zielgaskonzentration nicht mit ausreichender Aussagekraft aus dem Sensorsignal erfolgen kann, da dieses durch die Querempfindlichkeiten in unzulässiger Weise verfälscht ist.

Bisher musste die Verfälschung des Sensorsignals akzeptiert werden.
- Eine teilweise Behebung des Effektes kann durch eine intelligente der Applikation angepaßte Signalauswertung erreicht werden, was jedoch für viele Applikationen nur sehr eingeschränkt möglich ist.
- Alternativ kann ein zusätzlicher Sensor eingesetzt werden, der speziell auf das störende Gas sensitiv ist und dessen zusätzliches Signal zur Kompensation des Störeinflusses in einer entsprechenden Signalverarbeitung verwendet wird, was natürliche deutlich erhöhte Systemkosten bedingt.

Die Veröffentlichungen R. Paris et al.: "Low drift air-gap CMOS-FET gas sensor", PROCEEDINGS OF IEEE SENSORS 2002. ORLANDO, FL, JUNE 12 - 14, 2002, IEEE INTERNATIONAL CONFERENCE ON SENSORS, NEW YORK, NY : IEEE, US, Bd. VOL. 1 OF 2. CONF. 1, 12. Juni 2002 (2002-06-12), Seiten 421-425, ISBN: 0-7803-7454-1, M. Burgmair et al.: "Humidity and temperature compensation in work function gas sensor FETs", SENSORS AND ACTUATORS B, ELSEVIER SEQUOIA S.A., LAUSANNE, CH, Bd. 93, Nr. 1-2, 1. August 2003, Seiten 271-275, ISSN: 0925-4005, sowie Burgmair et al.:"Field effect transducers for work function gas measurements: device improvements and comparison of performance", SENSORS AND ACTUATORS B, ELESVIER SEQUOIA S:A:, LAUSANNE, CH, Bd. 95, Nr. 1-3, 15. Oktober 2003, Seiten 183-188, ISSN:0925-4005 und Z. Gergintschew et al.: "The capacitively controlled field effect transistor (CGFET) as a new low power gas sensor", SENSORS AND ACTUATORS B, ELESVIER SEQUOIA S:A:, LAUSANNE, CH, Bd. 36, Nr. 1, 1. Oktober 1996, Seiten 285-289, ISSN:0925-4005, betreffen einen Gassensor, bei dem die Kapazität der sensitiven Schicht ausgewertet wird.

Der Erfindung liegt die Aufgabe zugrunde bei FET-basierten Gassensoren eine Verfälschung des Sensorsignals durch Querempfindlichkeiten zu minimieren. Die Lösung dieser Aufgabe geschieht durch die Merkmalskombination der Ansprüche 1,5,6.

Vorteilhafte Ausgestaltungen können den Unteransprüchen entnommen werden.

Der Erfindung liegt die Erkenntnis zugrunde, dass durch den Einsatz eines FET-basierten Gassensors, bei dem durch eine entsprechende Ansteuerung nicht nur die Änderung der Austrittsarbeit (Grenzflächenpotentialänderung), sondern auch die Änderung der Kapazität der sensitiven Schicht ausgewertet wird, der Einfluss von Querempfindlichkeiten stark verringert wird.

Auf diese Weise werden zwei physikalisch unabhängige Signale aus der Schicht ausgelesen, die eine unterschiedliche Gassensitivität darstellen können.

Die Mechanismen, die erstens bei einer Reaktion mit Gasen eine Änderung der Austrittsarbeit und die zweitens eine Änderung der Kapazität der sensitiven Schicht bewirken sind weitgehend verschieden. Dadurch werden beide Größen eine unterschiedliche Gassensitivität zeigen. D.h. die Reaktion auf Zielgas und auf Störgas ist unterschiedlich. Wenn nun die Reaktionen auf beide Gase bekannt sind, kann der Einfluss des Störgases auf das Signal kompensiert und somit die Konzentration des Zielgases bestimmt werden. In einer nicht beanspruchten Ausführungsform alternativen Ausführungsform können auch beide Gaskonzentrationen berechnet werden.

Entsprechend der Erfindung liegen "zwei Sensoren in einem" vor, d.h. man hat durch die Betriebsweise zwei unabhängige Signale in einem Sensoraufbau generiert. Dies spart Kosten für einen zweiten Sensoraufbau. Zudem unterliegen Gassensoren im Langzeitbetrieb Drifteffekten. Hier neigen u.U. zwei separate Sensoraufbauten stärker zu unterschiedlichen Drifterscheinungen als ein Sensoraufbau, was die Fehlerkompensation in der Signalverarbeitung erschwert. Weitere Vorteile bestehen in zusätzlichen Informationen, die aus dem System auslesbar sind, wobei jedoch nur ein Sensoraufbau benötigt wird. Die Methode erlaubt ein "Zwei Sensoren in einem" Vorgehen.

Im Folgenden werden anhand von schematischen die Erfindung nicht einschränkenden Figuren Ausführungsbeispiele beschrieben.
- Figur 1: zeigt den Prinzipaufbau eines FET-Gassensors (CCFET-Typ),
- Figur 2: zeigt einen schematischen Aufbau von Austrittsarbeits-Gassensoren mit FET-Auslesung,
- Figuren 3 und 4: zeigen Gasreaktionen bei erfindungsgemäßen Mischausleseprinzip an einem FET.

### Für die Erfindung relevanter Sensoraufbau :

Geeignet für diese Vorgehensweise sind sowohl die klassischen Suspended gate FET Gassensoren (SGFET), mit einem Aufbau entsprechend Figur 2 ist, als auch die Gas FETs, bei denen die Kapazität durch die gassensitive Schicht und den Luftspalt ausgebildet wird und das elektrische Potential zu einem separat angebrachten Auslese FET über eine elektrisch leitfähige Verbindung übertragen wird (CCFET), entsprechend Figur 1.

### Ausführung der getrennten Auslesung von Kapazität und Grenzflächenpotential:

Bei der SGFET-Struktur entsprechend Figur 2 wird im beschriebenen Betrieb die Wirkung der Grenzflächenpotentiale auf die Kanalleitfähigkeit, Source-Drain-Strom, ausgelesen. Die Auslesung der Kapazität der sensitiven Schicht geschieht, indem an die Gateelektrode eine Wechselspannung (typisch 10-10000Hz) angelegt wird. Je nach Kapazität der sensitiven Schicht ändert sich die Einkopplung der Gatespannung auf die Kanalleitfähigkeit, d.h. der durch Wechselspannung am Gate bewirkte Wechselanteil des Source-Drain-Stroms hängt von der

Kapazität der sensitiven Schicht ab und ist damit ein direktes Maß für diese Kapazität.

Wenn aus spezifischen Gründen der Applikation die Gatespannung konstant gehalten wird, kann alternativ eine Wechselspannung an den Transistor selber, d.h. an den bulk-Anschluss des Si oder bei entsprechendem Aufbau an die Transistorwanne angelegt werden. Die Basisfunktion ist bei dieser Vorgehensweise die gleiche wie vorher beschrieben.

Bei einer CCFET-Struktur bei der noch eine weitere Elektrode, bezeichnet mit - capacitance weil -, angebracht unter dem Floating gate, vorhanden ist, kann die Wechselspannung auch wie oben beschrieben über die obere rückseitige Kontaktierung der sensitiven Schicht, sowie auch über den Transistor, angelegt werden. Besonders vorteilhaft kann bei dieser Gassensorvariante jedoch die Wechselspannung über die als capacitance weil bezeichnete Elektrode eingebracht werden. Diese Variante vermeidet sowohl ein zu starkes Verändern der Potentialverhältnisse im Luftspalt wie auch Beeinträchtigungen durch das Anlegen von Potentialen an den Transistor.

Dies gilt in ähnlicher Weise auch für die als FGFET bekannte Variante des SGFET, die in Figur 2 dargestellt ist.

### Für alle Varianten gilt:

- Die Anwendung der Wechselspannung zur Kapazitätsauslesung kann sowohl gleichzeitig mit der Auslesung des Grenzflächenpotentials erfolgen, wobei dann sowohl der Wechsel- wie auch Gleichanteil des Source-Drain-Stroms ausgelesen werden und auch eine Abwechslung zwischen beiden Betriebsmoden vorgesehen werden kann.
- Es ist nicht unbedingt die Verwendung einer Wechselspannung am Gate notwendig.
- Alternativ kann eine schnelle Änderung des Potentials erfolgen. Der Zeitverlauf der Wirkung dieser Potentialänderung auf den Source-Drain-Strom hängt hier ebenfalls von der Kapazität der sensitiven Schicht ab und kann genauso zu deren Bestinnung herangezogen werden.
- In einer nicht beanspruchten Ausführungsform kann alternativ auch die Transistorkennlinie, die Änderung des Source-Drain-Stroms mit der Gatespannung, ausgewertet werden. Da die sich daraus ergebende Transistorsteilheit auch durch die Luftspaltkapazitäten bestimmt wird, ist diese direkt abhängig von der Kapazität der sensitiven Schicht.

### Anwendung bei gassensitiven Materialien unterschiedlicher Morphologie:

Hier muss unterschieden werden zwischen porösen, also offen-porigen Materialien und kompakten, also dichten oder geschlossen porigen Materialien.

Bei porösen Materialien,
liegt oftmals ein starker Quereinfluss durch wechselnde Luftfeuchte vor. Dieser resultiert aus der Anlagerung der Feuchte an die Körner und bewirkt eine starke Veränderung der Kapazität der porösen Schicht.

Ein Beispiel hierfür ist das BaCO₃, welches als offenporige Schicht präpariert wird. Dieses ist gekennzeichnet durch
- eine Hauptempfindlichkeit des Sensormaterials auf CO₂, einem durch Austrittsarbeitsänderung an der äußeren Schichtbegrenzung entstehenden Potential, welches schichtdicken unabhängig,
- eine Querempfindlichkeit auf Feuchte, die durch Kapazitätsänderungen in den Poren der Schicht entsteht und daher linear abhängig ist von der Schichtdicke.

Bei einer gemischten Auslesung können Feuchteänderungen in unzulässiger Weise das Nutzsignal auf CO₂ verändern. Wenn nun gemäß der Erfindung die Kapazität separat ausgelesen wird, ist es möglich durch das separat gewonnene Feuchtesignal eine Korrektur des Messwertes vorzunehmen.

In vergleichbarer Weise können andere CO2-sensitive Materialien wie BaTiO3 oder auch mit CuO dotierte Materialvarianten oder alle anderen porösen Sensormaterialien vorteilhaft ausgewertet werden.

### Bei nicht porösen Materialien,

liegt dieser im wesentlichen Feuchteeffekte erzeugende Mechanismus natürlich nicht vor. Aber auch hier gibt es je nach Gasart und Detektions-Material unterschiedliche Wirkungen verschiedener Gase auf Austrittsarbeitsänderung und Kapazität. Erstere entsteht üblicherweise durch Grenzflächenreaktionen der Gase, letztere durch Reaktionen des Gases im Volumen der Sensorschicht.

Diese oben beschriebene Kapazitätsänderung kann z. B. durch Dickenänderung und/ oder Veränderung der Dielektrizitätskonstanten der sensitiven Schicht hervorgerufen werden.

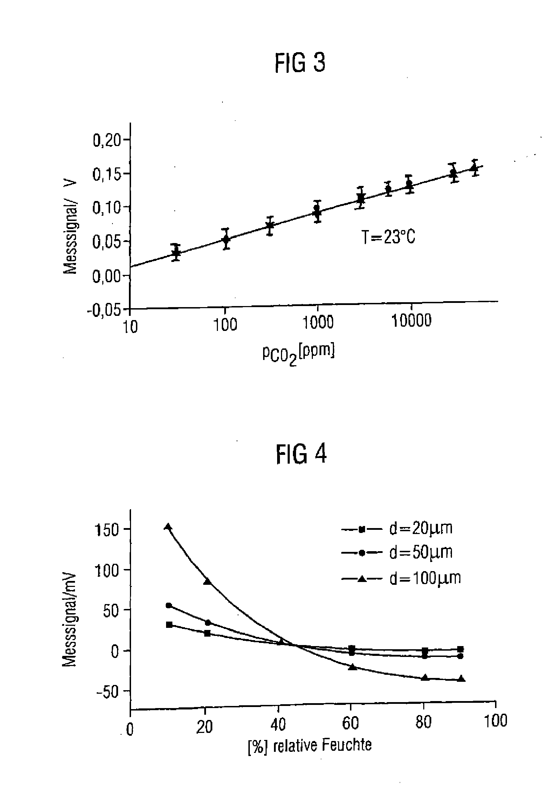

Die Figuren 3 und 4 zeigen Gasreaktionen bei erfindungsgemäßem Mischausleseprinzip an einem FET.

### Literatur

[I] Hybrider Flip-Chip Aufbau zum kostengünstigen Aufbau von Austrittsarbeits-Gassensoren, Deutsche Patentanmeldung DE 19814857 A1,
[II] Aufbau des in hybrider flip-chip technologie verwirklichten Feldeffektransistors, Deutsche Patentanmeldung, DE 19956744 A1,
[III] Gasdetektion nach dem Prinzip der Messung von Austrittsarbeiten, Deutsche Patentanmeldung DE 19849932 A1,

## Patentansprüche

1. Verfahren zur Minimierung von Querempfindlichkeiten bei FET- basierten Gassensoren, deren Sensorsignal durch die Änderung der Austrittsarbeit an einer sensitiven Schicht generiert wird, wobei zusätzlich zur Auslesung der Änderungen der Austrittsarbeit die Änderung der Kapazität der sensitiven Schicht ausgewertet wird, und wobei die beiden Größen eine unterschiedliche Reaktion auf ein Zielgas und ein Störgas aufzeigen,
**dadurch gekennzeichnet, dass**
zur Auslesung der Kapazität der sensitiven Schicht an die Gate-Elektrode eines SGFET oder eines CCFET oder eines FGFET eine zeitlich sich ändernde Spannung angelegt wird, wodurch der Source/Drain-Strom einen Wechselanteil aufweist, der von der Kapazität der sensitiven Schicht abhängig ist und aus der bekannten unterschiedlichen Reaktion, der Einfluss des Störgases auf das Sensorsignal kompensiert wird.

2. Verfahren nach Anspruch 1 bei dem die zeitlich sich ändernde Spannung eine Wechselspannung ist.

3. Verfahren nach Anspruch 1, bei dem der Zeitverlauf der Potentialänderung auf den Source / Drain-Strom zur Ermittlung der Kapazität der sensitiven Schicht verwendet wird.

4. Verfahren nach Anspruch 3, bei dem die Änderung des Source/Drain-Stromes mit der Gatespannung zur Ermittlung der Kapazität der sensitiven Schicht verwendet wird.

5. Verfahren zur Minimierung von Querempfindlichkeiten bei FET- basierten Gassensoren, deren Sensorsignal durch die Änderung der Austrittsarbeit an einer sensitiven Schicht generiert wird, wobei zusätzlich zur Auslesung der Änderungen der Austrittsarbeit die Änderung der Kapazität der sensitiven Schicht ausgewertet wird, und wobei die beiden Größen eine unterschiedliche Reaktion auf ein Zielgas und auf ein Störgas aufzeigen,
**dadurch gekennzeichnet, dass**
zur Auslesung der Kapazität der sensitiven Schicht unter Konstanthaltung der Gatespannung eine Wechselspannung an einen Bulk-Anschluss oder an eine Transistorwanne des Transistors angelegt wird, wodurch der Source/Drain-Strom einen Wechselanteil aufweist, der von der Kapazität der sensitiven Schicht abhängig ist, und
aus der bekannten unterschiedlichen Reaktion, der Einfluss des Störgases auf das Sensorsignal kompensiert wird.

6. Verfahren zur Minimierung von Querempfindlichkeiten bei FET- basierten Gassensoren, deren Sensorsignal durch die Änderung der Austrittsarbeit an einer sensitiven Schicht generiert wird, wobei zusätzlich zur Auslesung der Änderungen der Austrittsarbeit die Änderung der Kapazität der sensitiven Schicht ausgewertet wird, und wobei die beiden Größen eine unterschiedliche Reaktion auf ein Zielgas und auf ein Störgas aufzeigen,
**dadurch gekennzeichnet, dass**
zur Auslesung der Kapazität der sensitiven Schicht unter Konstanthaltung der Gatespannung eine Wechselspannung bei einer CCFET-Struktur über eine obere rückseitige Kontaktierung der sensitiven Schicht oder über eine weitere als capacitance well ausgebildete Elektrode angelegt wird, wodurch der Source/Drain-Strom einen Wechselanteil aufweist, der von der Kapazität der sensitiven Schicht abhängig ist, und
aus der bekannten unterschiedlichen Reaktion, der Einfluss des Störgases auf das Sensorsignal kompensiert wird.

7. Verfahren nach einem der vorhergehenden Ansprüche, bei dem die Querempfindlichkeit auf Feuchtigkeit eliminiert wird.

## Claims

1. Method of minimising cross-sensitivities in FET-based gas sensors, the sensor signal of which is generated by change in the work function of a sensitive layer, wherein the change in the capacitance of the sensitive layer is evaluated in addition to reading out the changes in the work function and wherein the two variables exhibit a different reaction to an objective gas and a disturbing gas, **characterised in that** for reading out the capacitance of the sensitive layer a voltage changing over time is applied to the gate electrode of an SGFET or a CCFET or an FGFET, whereby the source/drain current has an alternating component dependent on the capacitance of the sensitive layer and compensation for the influence of the disturbing gas on the sensor signal is provided from the known different reaction.

2. Method according to claim 1, in which the voltage changing over time is an alternating voltage.

3. Method according to claim 1, in which the time plot of the change in potential on the source/drain current is used for determining the capacitance of the sensitive layer.

4. Method according to claim 3, in which the change in the drain/source current by the gate voltage is used for determining the capacitance of the sensitive layer.

5. Method of minimising cross-sensitivities in FET-based gas sensors, the sensor signal of which is generated by change in the work function of a sensitive layer, wherein the change in the capacitance of the sensitive layer is evaluated in addition to reading out the changes in the work function and wherein the two variables exhibit a different reaction to an objective gas and a disturbing gas, **characterised in that** for reading out the capacitance of the sensitive layer while keeping the gate voltage constant an alternating voltage is applied to a bulk terminal or a transistor trough of the transistor, whereby the source/drain current has an alternating component dependent on the capacitance of the sensitive layer and compensation for the influence of the disturbing gas on the sensor signal is provided from the known different reaction.

6. Method of minimising cross-sensitivities in FET-based gas sensors, the sensor signal of which is generated by change in the work function of a sensitive layer, wherein the change in the capacitance of the sensitive layer is evaluated in addition to reading out the changes in the work function and wherein the two variables exhibit a different reaction to an objective gas and a disturbing gas, **characterised in that** for reading out the capacitance of the sensitive layer while keeping the gate voltage constant an alternating voltage is in the case of a CCFET structure applied by way of upper rear-side contact-making with the sensitive layer or by way of a further electrode formed as a capacitance well, whereby the source/drain current has an alternating component dependent on the capacitance of the sensitive layer and compensation for the influence of the disturbing gas on the sensor signal is provided from the known different reaction.

7. Method according to any one of the preceding claims, in which the cross-sensitivity to moisture is eliminated.

## Revendications

1. Procédé pour minimiser des sensibilités transversales dans des capteurs de gaz à base de transistors à effet de champ, dont le signal de capteur est généré par la modification du travail de sortie à une couche sensible, dans lequel en plus de la lecture des modifications du travail de sortie on évalue la modification de la capacité de la couche sensible, et dans lequel les deux grandeurs révèlent une réaction différente à un gaz cible et à un gaz perturbateur, **caractérisé en ce que**
pour la lecture de la capacité de la couche sensible, on applique à l'électrode de grille d'un SGFET ou d'un CCFET ou d'un FGFET une tension variant dans le temps, le courant source/drain présentant ainsi une partie alternative, qui dépend de la capacité de la couche sensible, et on compense l'influence du gaz perturbateur sur le signal de capteur à partir de la réaction différente connue.

2. Procédé selon la revendication 1, dans lequel la tension variant dans le temps est une tension alternative.

3. Procédé selon la revendication 1, dans lequel on utilise l'évolution temporelle de la variation de potentiel sur le courant source/drain pour déterminer la capacité de la couche sensible.

4. Procédé selon la revendication 3, dans lequel on utilise la modification du courant source/drain avec la tension de grille pour déterminer la capacité de la couche sensible.

5. Procédé pour minimiser des sensibilités transversales dans des capteurs de gaz à base de transistors à effet de champ, dont le signal de capteur est généré par la modification du travail de sortie à une couche sensible, dans lequel en plus de la lecture des modifications du travail de sortie on évalue la modification de la capacité de la couche sensible, et dans lequel les deux grandeurs révèlent une réaction différente à un gaz cible et à un gaz perturbateur, caractérisé en ce quepour la lecture de la capacité de la couche sensible, en maintenant constante la tension de grille, on applique une tension alternative à un raccord de masse ou à une cuvette de transistor du transistor, le courant source/drain présentant ainsi une partie alternative, qui dépend de la capacité de la couche sensible, et on compense l'influence du gaz perturbateur sur le signal de capteur à partir de la réaction différente connue.

6. Procédé pour minimiser des sensibilités transversales dans des capteurs de gaz à base de transistors à effet de champ, dont le signal de capteur est généré par la modification du travail de sortie à une couche sensible, dans lequel en plus de la lecture des modifications du travail de sortie on interprète la modification de la capacité de la couche sensible, et dans lequel les deux grandeurs révèlent une réaction différente à un gaz cible et à un gaz perturbateur, **caractérisé en ce que** pour la lecture de la capacité de la couche sensible, en maintenant constante la tension de grille, on applique une tension alternative pour une structure CCFET par une mise en contact arrière supérieure de la couche sensible ou par une autre électrode réalisée sous la forme d'un puits de capacité, le courant source/drain présentant ainsi une partie alternative, qui dépend de la capacité de la couche sensible, et on compense l'influence du gaz perturbateur sur le signal de capteur à partir de la réaction différente connue.

7. Procédé selon l'une quelconque des revendications précédentes, dans lequel la sensibilité transversale à l'humidité est éliminée.
